# EUROPEAN PATENT SPECIFICATION

(11) **EP 3 911 284 B1**
(45) Date of publication and mention of the grant of the patent: **01.11.2023**
(21) Application number: 19909769.2
(22) Date of filing: 03.07.2019
(51) Int. Cl.: A61F 9/007

(54) **SURGICAL INSTRUMENT FOR GONIOTOMY PROCEDURE AND METHOD OF MANUFACTURE**
CHIRURGISCHES INSTRUMENT FÜR GONIOTOMIE UND VERFAHREN ZUR HERSTELLUNG
INSTRUMENT CHIRURGICAL POUR PROCÉDURE DE GONIOTOMIE ET PROCÉDÉ DE FABRICATION

(30) Priority: 14.01.2019 US 201962792058 P
(43) Date of publication of application: 24.11.2021
(73) Proprietor: Raico International, LLC, Willowbrook, IL 60527 (US)
(72) Inventor: YOKOYAMA, Mitsunobu, Knowledge Square, 1F 7300053 (JP)
(74) Representative: König Szynka Tilmann von Renesse Patentanwälte Partnerschaft mbB München
(86) International application number: PCT/US2019/040538
(87) International publication number: WO 2020/149877

(56) References cited:
- US-A- 5 921 998
- US-A1- 2002 151 769
- US-A1- 2006 276 759
- US-A1- 2007 282 348
- US-A1- 2007 282 348
- US-A1- 2018 193 191
- US-A1- 2018 193 191

## Description

### FIELD OF THE INVENTION

The present invention relates to a surgical instrument for performing ophthalmological procedures for treatment of eye diseases, such as glaucoma, and more particularly to a goniotomy surgical instrument having a tip portion with an elastomeric element having a roughened, abrasive surface but which is soft to the touch.

### BACKGROUND OF THE INVENTION

A goniotomy is a surgical procedure primarily used to treat congenital glaucoma. It is caused by a developmental arrest of some of the structures within the anterior (front) segment of the eye. These structures include the iris and the ciliary body, which produces the aqueous fluid needed to maintain the integrity of the eye. These structures do not develop normally in the eyes of patients with isolated congenital glaucoma. Instead, they overlap and block the trabecular meshwork, which is the primary drainage system for the aqueous fluid. As a result of this blockage, the trabecular meshwork itself becomes thicker and the drainage holes within the meshwork are narrowed. These changes lead to an excess of fluid in the eye, which can cause increased pressure that can damage the internal structures of the eye and cause glaucoma.

The purpose of a goniotomy is to clear the obstruction to aqueous outflow from the eye, which in turn lowers the intraocular pressure (IOP). Lowering the lOP helps to stabilize the enlargement of the cornea and the distension and stretching of the eye that often occur in congenital glaucoma. The size of the eye, however, will not return to normal. Most importantly, once the aqueous outflow improves, damage to the optic nerve is halted or reversed. The patient's visual acuity may improve after surgery.

The goniotomy procedure can restore normal drainage of aqueous humor from the eye by removing a full thickness segment of the trabecular meshwork, thus allowing the aqueous humor to drain through the open area from which the strip of trabecular meshwork has been removed. The goniotomy procedure and certain prior art instruments useable to perform such procedure are described In U.S. Patent No. 6,979,328 and U.S. Patent Application Publication No. US 2018/0289544 A1.

From the disclosure of US 2007/282348 A1 an ophthalmic microsurgical instrument for scraping or selectively removing tissue membrane from the retina, or other areas of the eye is known. Latter microsurgical instrument includes a handle for grasping of the instrument by an ophthalmic surgeon, an elongated rigid tube having one end rigidly attached to the handle, and its other end secured to a solid resilient tip. The solid resilient tip supports an abrasive element for contacting the portions of the thin tissue to be removed from the retina and other areas of the eye. Herewith described disclosure teaches that said tip may be tapered or pointed and may be formed by cutting the corresponding rod at a bevel with a given angle relative to the longitudinal axis.

At present there remains a need in the art for the development of simple, inexpensive and accurate instruments useable to perform the procedure of abrading and/or scraping the trabecular meshwork in the eye.

### SUMMARY OF THE INVENTION

In accordance with the present invention, a surgical instrument is disclosed which is particularly configured to facilitate performing a goniotomy such as for the treatment of glaucoma. The instrument includes a hand grip portion having an elongated configuration, with a proximal end and a distal end. The instrument further includes a tip portion which extends from, and which is operably connected with, the distal end of the hand grip portion. The tip portion has the form of an elastomeric element having a base that is secured to the distal end of the hand grip portion and a free, cantilevered end extending from the base. The free end has a relatively abrasive surface for engaging the eye.

In another aspect of the present invention, the elastomeric element defines a central axis and the free end is configured to taper inwardly toward the central axis, in a direction away from the base.

In one aspect of the present invention, the relatively abrasive surface of the free end comprises an impregnated abrasive composition. In one presently preferred form of the invention, the abrasive composition comprises diamond particles. In another preferred form of the invention, the relatively abrasive surface of the free end has the form of surface-roughening.

In still another form of the present invention, the hand grip portion includes proximal and distal sections arranged at an obtuse angle (α) relative to each other, preferably between about 120 and about 140 degrees.

In another aspect of the present invention, the elastomeric element is silicone. Preferably, the silicone has a durometer of between about 60 and about 90 on the Shore 00 scale.

In still another form of the present invention, the elastomeric element includes (i) an upper concave portion that is substantially smooth, and (ii) an opposite, lower convex portion that includes the relatively abrasive surface.

In another broad form of the present invention, a method of making a surgical instrument is disclosed. The method includes the step of forming a hand grip portion having an elongated configuration, with proximal and distal ends. The method includes the step of securing a tip portion to extend from the distal end of the hand grip portion. The tip portion is an elastomeric element having a base secured to the distal end of the hand grip portion, and a free end extending from the base. The method includes the step of providing the free end with a relatively abrasive surface.

According to another aspect of the present invention, the step of providing the relatively abrasive surface includes surface roughening of at least a portion of the free end of the elastomeric element.

According to another aspect of the present invention, the step of providing the relatively abrasive surface includes impregnating the free end with abrasive particles.

According to another aspect of the present invention, the method further includes the step of forming the free end with one of a frusto-conical configuration, pyramidal configuration, or arcuate configuration.

### BRIEF DESCRIPTION OF THE DRAWINGS

In the accompanying drawings forming part of the specification, in which like numerals are employed to designate like parts throughout the same,
FIG. 1 is a fragmentary, side elevation view of a first embodiment of a surgical instrument not part of the claimed invention but illustrating the technological background, and FIG. 1 shows only a distal portion of the instrument;
FIG. 2 is an enlarged, detailed side elevation view of the portion of the instrument circled in FIG. 1, and FIG. 2 shows the operative, distal end of the instrument;
FIG. 3 is an enlarged, fragmentary, side elevation view of a second embodiment of a surgical instrument according to the present invention, and FIG. 5 shows only a distal, operative portion of the instrument;
FIG. 4 is an enlarged, fragmentary, perspective view taken from above of the portion of the instrument illustrated in FIG. 3;
FIG. 5 is a fragmentary, side elevation view of a third embodiment of a surgical instrument not part of the claimed invention but illustrating the technological background;
FIG. 6 is an enlarged, detailed side elevation view of the portion of the instrument circled in FIG. 5, and FIG. 6 shows the operative, distal end of the instrument;
FIG. 7 is an enlarged, detailed bottom view of the portion of the instrument circled in FIG. 5;
FIG. 8 is an enlarged, fragmentary, perspective view taken from above of the portion of the instrument illustrated in FIG. 6; and
FIG. 9 is an enlarged, fragmentary, perspective view taken from the front of the portion of the instrument illustrated in FIG. 6.

### DESCRIPTION OF THE PREFERRED EMBODIMENTS

While this invention is susceptible of embodiment in many different forms, this specification and the accompanying drawings disclose only specific forms as examples of the invention. The invention is not intended to be limited to only the embodiments so described, and the scope of the invention will be pointed out in the appended claims.

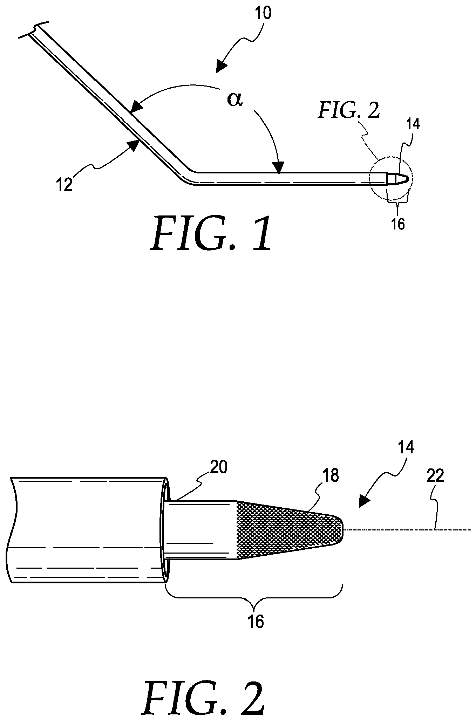

A first embodiment of a goniotomy surgical instrument is illustrated in FIGS. 1 and 2, wherein the instrument is designated generally by the reference number 10. Said first embodiment shown in FIGS. 1 and 2 is not part of the claimed invention. The first illustrated embodiment of the instrument 10 includes an elongated hand grip portion 12 for being gripped by a user of the instrument 10, and which can be provided with either a rounded configuration as illustrated or with a flattened, polygonal, or irregular configuration (not illustrated). The hand grip portion 12 has a proximal end and a distal end.

Referring to FIG. 1, the hand grip portion 12 proximal and distal sections or portions are arranged at an obtuse angle (α) relative to each other of between about 120 degrees and about 140 degrees.

The instrument 10 includes a specifically configured scraping or abrading tip portion 14, extending from the distal end of the hand grip portion 12, which facilitates scraping of the trabecular meshwork to permit drainage of aqueous humor to enhance the vision of the patient.

Notably, the tip portion 14 comprises an elastomeric element 16 having a base 20 secured to the distal end of the hand grip portion 12, and free end 18 extending from the base 20. In accordance with the invention, the free end 18 has a relatively abrasive surface.

With reference to FIG. 2, it can be seen that the free end 18 tapers inwardly (e.g., narrows) in a direction away from the base 20 toward a central axis 22. The free end 18 of the elastomeric element 16 has a frustoconical configuration while the base 20 of the elastomeric element 16 has a cylindrical configuration. The cylindrical base 20 is configured without the abrasive surface provided on or in the free end 18.

In one preferred form of the first embodiment of the instrument 10, the elastomeric element 16 has a total length extending from the handle 12 in the direction along the central axis 22 of about 1.7 mm, with the base 20 having a length of about 0.7 mm and the free end 18 having a length of about 1.0 mm.

In another presently preferred form of the first embodiment of the instrument 10, the elastomeric element 16 has a total length extending from the handle 12 in the direction along the central axis 22 of about 1.2 mm, with the base 20 having a length of about 0.5 mm and the free end 18 having a length of about 0.7 mm.

In order to effect the desired scraping/abrading action of the instrument 10, the relatively abrasive surface of the free end 18 comprises an impregnated abrasive composition. The impregnated abrasive composition may comprise diamond particles or abrasive granular or dust particles. Alternatively, the abrasive surface may be provided through subjecting the elastomeric element 16 to a secondary coating process. In yet another alternative, the relatively abrasive surface of the free end 18 is formed from a surface-roughening or texturing of the elastomeric material of the element 16.

In one presently preferred form of the invention, the free end 18 of the instrument has a surface roughness of between about N8-N12 according to the ISO 1302 Roughness Grade Numbers. In the preferred form, the free end 18 of the tip portion 14 is provided with a frusto-conical configuration when the elastomeric element 16 is formed. Preferably, the elastomeric element 16 is formed from a medical grade silicone material with a durometer of between about 60 and about 90 on the Shore 00 scale, while the hand grip portion 12 of the instrument is formed from a metal or sufficiently stiff plastic or composite material.

The handle portion 12 of the instrument 10 is preferably made of material like malleable nitinol or other malleable alloys or metals and is provided with a tip portion 14 in the form of an elastomeric element 16 having a base 20, secured to the distal end of the hand grip portion 12, and a free end 18 extending from the base 20. Notably, the free end 20 is provided with a relatively abrasive surface, compared to the material of the elastomeric element 16. The elastomeric element 16 may be secured to the handle portion 12 of the instrument 10 by mechanical fit, overmolding or bi-injection molding, adhesive, welding, or any other suitable means.

The inventor of the present invention has found that using an instrument as described herein having a relatively abrasive surface may be sufficient to restore normal or sufficient drainage through the trabecular meshwork with the abrasion of the clogged or blocked areas of the eye.

In one preferred method of operation of the instrument 10, the user would create two incisions or ports within the eye. The ports are used to accommodate the insertion of the instrument 10, and the implantation and removal of viscoelastic substances (OVD) from the eye, such as with an irrigation/aspiration instrument or instruments. The ports would be used to fill the anterior chamber of the eye with viscoelastic material to the appropriate viscosity. The instrument 10 into the first port to gently rub the area of the trabecular meshwork, about 120 degrees, with the free end 18 of the tip 14 about three to five times to remove deposits. A MORI Goniotomy lens may be used to observe the area being rubbed. The ports would be used to remove the viscoelastic material from the anterior chamber of the eye. The anterior chamber may be filled with balanced salt solution (BSS) to reach the desired intraocular pressure. In one preferred form of the method, the side ports in the eye are formed at the 12 o'clock and 3 o'clock positions. In another preferred form of the method of use of the instrument 10, the side ports in the eye are formed at the 10 o'clock and 3 o'clock positions, and a third port is formed at the 12 o'clock position to accommodate insertion of an intraocular lens (IOL).

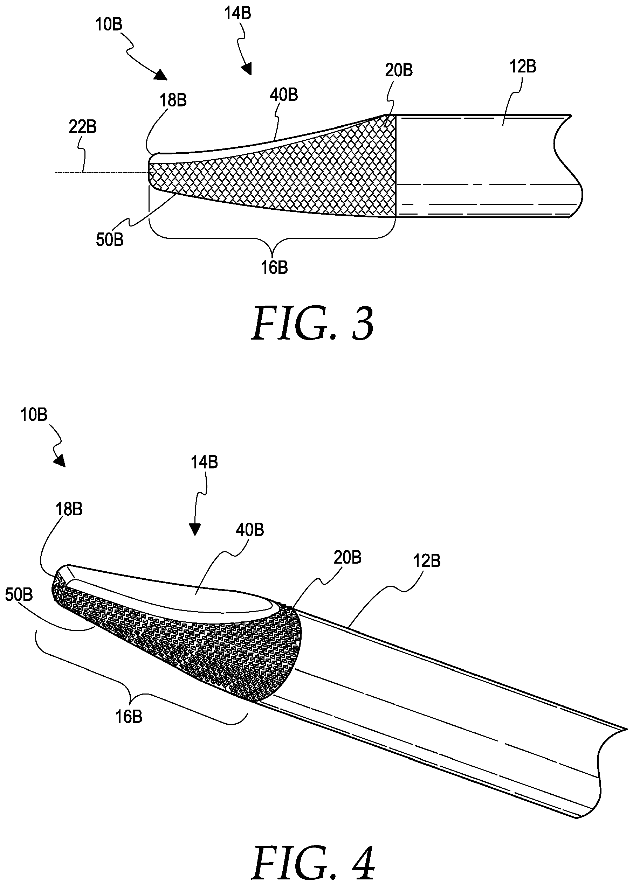

A second embodiment of a goniotomy surgical instrument of the present invention is illustrated in FIGS. 3 and 4, wherein the instrument is designated generally by the reference number 10B. The numbered features of the third embodiment of the instrument 10B illustrated in FIGS. 3 and 4 are designated generally with the suffix letter "B" and are analogous to features of the first embodiment of the instrument 10 that share the same number (without the suffix letter "B").

The second illustrated embodiment of the instrument 10B is similar in nature to the first illustrated embodiment of the instrument 10 and includes a hand grip portion 12B having a proximal and distal end with a tip portion 14B extending from the distal end of the hand grip portion 12B. The tip portion 14B comprises an elastomeric element 16B having a base 20B secured to the distal end of the hand grip portion 12B, and a free end 18B extending from the base 20B along a central axis 22B.

With reference to FIG. 3, the instrument 10B differs from the previously-discussed embodiment of the instrument 10, in that the instrument 10B has an elastomeric element 16B with a different tapering configuration that is currently understood to better conform to the inside and outside of the Schlemm's canal. Specifically, the elastomeric element 16B includes an upper concave portion 40B that is substantially free of any abrasive or roughened surface. The elastomeric element 16B further includes an opposite, lower convex portion 50B that has an abrasive or roughened surface.

With reference to FIG. 4, the abrasive surface forms a band that extends generally around the lateral sides and bottom of the elastomeric element 16B. The top side beyond the band is substantially free of any roughened or abrasive surface.

The inventor has found that the instrument 10B having an abrasive surface extending substantially along the lateral and bottom surfaces of the elastomeric element 16B, is particularly suited for restoring the normal or sufficient drainage through the trabecular meshwork with the abrasion of the clogged or blocked areas of the eye, while presenting only the non-abrasive surfaces against the endothelium.

In one preferred form of the second illustrated embodiment of the inventive instrument 10B, the elastomeric element 16B has a total length extending from the handle 12B in the direction along the central axis 22B of about 1.0 mm, with the base 20B having a diameter or width of about 0.23 mm and the free end 18B having a diameter or width of about 0.1 mm. The upper concave portion 40B that is substantially free of any abrasive or roughened surface has a radius of about 2.56 mm. The lower convex portion 50B has a radius of about 3.0 mm.

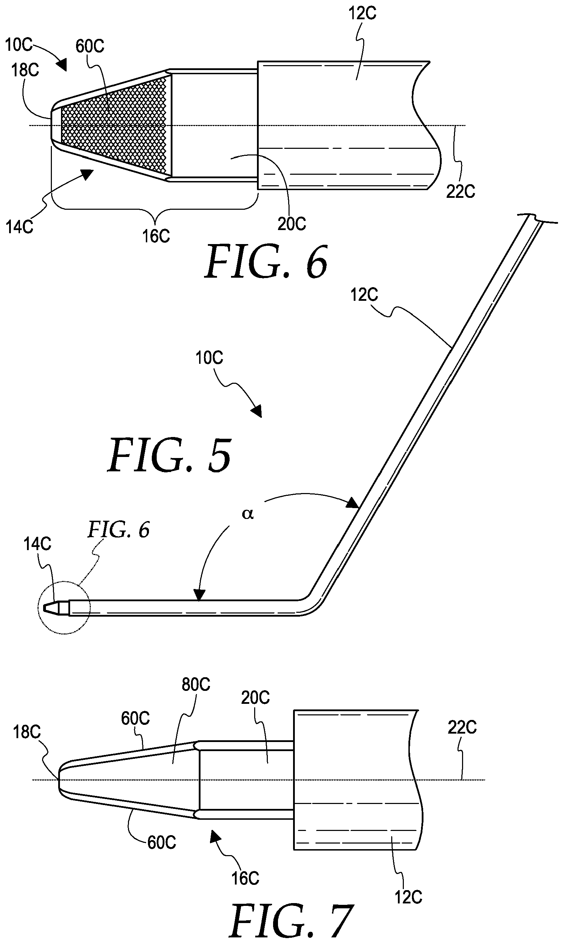

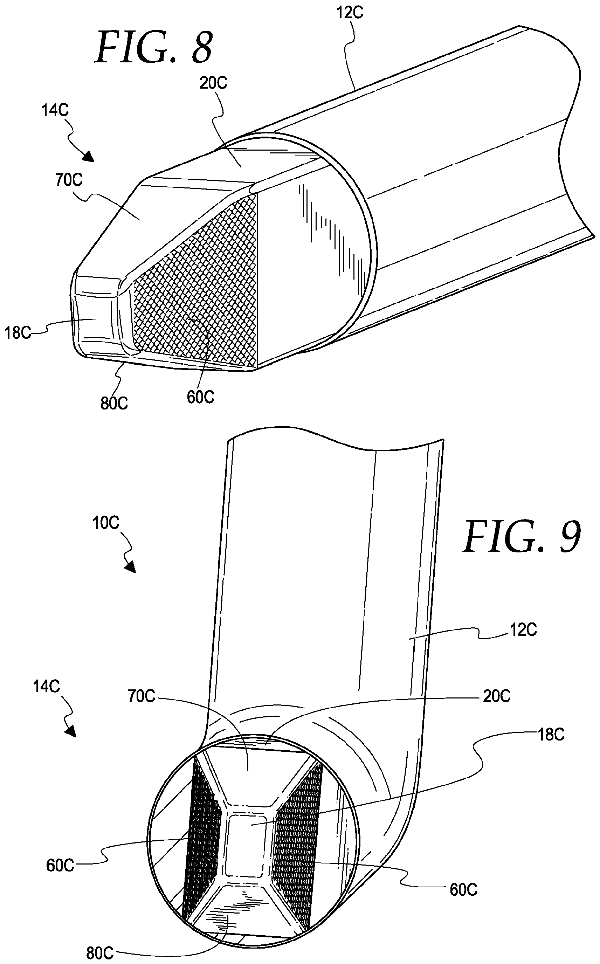

A third embodiment of a goniotomy surgical instrument is illustrated in FIGS. 5-9, wherein the instrument is designated generally by the reference number 10C. Said third embodiment shown in FIGS. 5-9 is not part of the claimed invention The numbered features of the third embodiment of the instrument 10C illustrated in FIGS. 5-9 are designated generally with the suffix letter "C" and are analogous to features of the first embodiment of the instrument 10 that share the same number (without the suffix letter "C").

The third illustrated embodiment of the instrument 10C is similar in nature to the first illustrated embodiment of the instrument 10 and includes a hand grip portion 12C having a proximal and distal end with a tip portion 14B extending from the distal end of the hand grip portion 12C. The tip portion 14C comprises an elastomeric element 16C having a base 20C secured to the distal end of the hand grip portion 12C, and a free end 18C extending from the base 20C along a central axis 22C.

With reference to FIG. 6, the instrument 10C differs from the previously-discussed embodiment of the instrument 10, in that the instrument 10C has an elastomeric element 16C with a different tapering configuration that is currently understood to conform to the inside and outside dimensions of the Schlemm's canal. Specifically, the elastomeric element 16C includes a pyramidal configuration with opposing, lateral sides 60C that each have an abrasive or roughened surface. The other sides (top, bottom, and/or end) are substantially free of any abrasive or roughened surface.

The inventor has found that the instrument 10C having an abrasive surface extending only along the lateral surfaces of the elastomeric element 16C, is particularly suited for restoring the normal or sufficient drainage through the trabecular meshwork with the abrasion of the clogged or blocked areas of the eye, while presenting only the non-abrasive surfaces against the endothelium.

In one preferred form of the third illustrated embodiment of the instrument 10C, the elastomeric element 16C has a total length extending from the handle 12C in the direction along the central axis 22C of about 1.0 mm, with the base 20C having a width (normal to the axis 22C) of about 0.33 mm and a length along the axis 22C of about 0.39 mm. The free end 18C has a width of about 0.2 mm, and a length along the axis 22C of about 0.6 mm. The internal angle α of the instrument handle 12C is preferably about 120 degrees.

The inventor has further determined that it may further be advantageous to combine any one of the instruments disclosed herein with an irrigation system and an aspiration system. For example, the instruments may be incorporated into an aspiration/irrigation handpiece to supply irrigating liquids to the eye in order to aid in flushing and aspirating dislodged particles in the eye during the use of the instruments.

It will further be understood that the instruments disclosed herein may be incorporated into a larger machine or device, whereby the hand grip portion is connected to such a machine or device, and may be controlled, operated, or manipulated by such a machine or device and not necessarily by hand.

Other features and advantages will be readily apparent from the following the accompanying drawings and the appended claims.

## Claims

1. A surgical instrument (10B), comprising:
a hand grip portion (12B) having an elongated configuration, having proximal and distal ends; and
a tip portion (14B) extending from the distal end of said hand grip portion (12B),
said tip portion (14B) comprising an elastomeric element (16B) having a base (20B) secured to said distal end of said hand grip portion (12B), and a free end (18B) extending from said base (20B), said free end (18B) having a relatively abrasive surface;
wherein said elastomeric element (16B) includes (i) an upper concave portion (40B) that is substantially smooth, and (ii) an opposite, lower convex portion (50B) that includes said relatively abrasive surface.

2. A surgical instrument (10B) in accordance with claim 1, wherein said free end (18B) tapers inwardly in a direction away from said base (20B).

3. A surgical instrument (10B) in accordance with claim 1, wherein said relatively abrasive surface of said free end (18B) comprises an impregnated abrasive composition.

4. A surgical instrument (10B) in accordance with claim 3, wherein said abrasive composition comprises diamond particles.

5. A surgical instrument (10B) in accordance with claim 3, wherein said relatively abrasive surface of said free end (18B) comprises surface-roughening.

6. A surgical instrument (10B) in accordance with claim 1, wherein said hand grip portion (12B) comprises proximal and distal section arranged at an obtuse angle (α) to each other.

7. A surgical instrument (10B) in accordance with claim 6, wherein said obtuse angle (α) is between about 120 and about 140 degrees.

8. A surgical instrument (10B) in accordance with claim 1, wherein elastomeric element (16B) is silicone.

9. A method of making a surgical instrument (10B), comprising the steps of:
forming a hand grip portion (12B) having an elongated configuration, having proximal and distal ends;
securing a tip portion (14B) to extend from said distal end of said hand grip portion (12B), said tip portion (12B) comprising an elastomeric element (16B) having a base (20B) secured to said distal end of said hand grip portion (12B), and free end (18B) extending from said base (20B);
providing said free end (18B) with a relatively abrasive surface; and
wherein said elastomeric element (16B) includes (i) an upper concave portion (40B) that is substantially smooth, and (ii) an opposite, lower convex portion (50B) that includes said relatively abrasive surface.

10. A method of making a surgical instrument (10B) in accordance with claim 9, wherein said step of providing said relatively abrasive surface comprises
surface roughening of at least a portion of said free end (18B) of said elastomeric element (16B); or
impregnating said free end (18B) with abrasive particles; or
roughening only lateral portions of said free end (18B).

## Patentansprüche

1. Chirurgisches Instrument (10B), umfassend:
einen Handgriffabschnitt (12B) aufweisend eine längliche Konfiguration, welcher ein proximales und ein distales Ende aufweist; und
einen Spitzenabschnitt (14B), welcher sich von dem distalen Ende des Handgriffabschnitts (12B) erstreckt,
wobei der Spitzenabschnitt (14B) ein elastomeres Element (16B) aufweisend eine Basis (20B), welche an dem distalen Ende des Handgriffabschnitts (12B) fixiert ist, und ein freies Ende (18B), welches sich von der Basis (20B) erstreckt, umfasst, wobei das freie Ende (18B) eine relativ abrasive Oberfläche aufweist;
wobei das elastomere Element (16B) (i) einen oberen konkaven Abschnitt (40B), welcher im Wesentlichen glatt ist, und (ii) einen entgegengesetzten, unteren konvexen Abschnitt (50B), welcher die relativ abrasive Oberfläche beinhaltet, beinhaltet.

2. Chirurgisches Instrument (10B) nach Anspruch 1, wobei sich das freie Ende (18B) einwärts in einer Richtung weg von der Basis (20B) verjüngt.

3. Chirurgisches Instrument (10B) nach Anspruch 1, wobei die relativ abrasive Oberfläche des freien Endes (18B) eine imprägnierte abrasive Zusammensetzung umfasst.

4. Chirurgisches Instrument (10B) nach Anspruch 3, wobei die abrasive Zusammensetzung Diamantpartikel umfasst.

5. Chirurgisches Instrument (10B) nach Anspruch 3, wobei die relativ abrasive Oberfläche des freien Endes (18B) eine Oberflächenaufrauhung umfasst.

6. Chirurgisches Instrument (10B) nach Anspruch 1, wobei der Handgriffabschnitt (12B) einen proximalen und einen distalen Bereich umfasst, welche in einem stumpfen Winkel (α) zueinander angeordnet sind.

7. Chirurgisches Instrument (10B) nach Anspruch 6, wobei der stumpfe Winkel (α) zwischen etwa 120 und etwa 140 Grad ist.

8. Chirurgisches Instrument (10B) nach Anspruch 1, wobei das elastomere Element (16B) Silikon ist.

9. Verfahren zur Herstellung eines chirurgischen Instruments (10B), umfassend die Schritte:
Ausbilden eines Handgriffabschnitts (12B) aufweisend eine längliche Konfiguration, welcher ein proximales und ein distales Ende aufweist;
Fixieren eines Spitzenabschnitts (14B), um sich von dem distalen Ende des Handgriffabschnitts (12B) zu erstrecken, wobei der Spitzenabschnitt (14B) ein elastomeres Element (16B) aufweisend eine Basis (20B), welche an dem distalen Ende des Handgriffabschnitts (12B) fixiert ist, und ein freies Ende (18B), welches sich von der Basis (20B) erstreckt, umfasst;
Bereitstellen des freien Endes (18B) mit einer relativ abrasiven Oberfläche; und
wobei das elastomere Element (16B) (i) einen oberen konkaven Abschnitt (40B), welcher im Wesentlichen glatt ist, und (ii) einen entgegengesetzten, unteren konvexen Abschnitt (50B), welcher die relativ abrasive Oberfläche beinhaltet, beinhaltet.

10. Verfahren zur Herstellung eines chirurgischen Instruments (10B) nach Anspruch 9, wobei der Schritt des Bereitstellens der relativ abrasiven Oberfläche umfasst
Aufrauen der Oberfläche von mindestens einem Abschnitt des freien Endes (18B) des elastomeren Elements (16B); oder
Imprägnieren des freien Endes (18B) mit abrasiven Partikeln; oder
Aufrauen nur seitlicher Abschnitte des freien Endes (18B).

## Revendications

1. Instrument chirurgical (10B), comprenant :
une partie de préhension (12B) de configuration allongée et présentant des extrémités proximale et distale; et
une partie de pointe (14B) s'étendant à partir de l'extrémité distale de ladite partie de préhension (12B);
ladite partie de pointe (14B) comprenant un élément élastomère (16B) présentant une base (20B) fixée à ladite extrémité distale de ladite partie de préhension (12B), et une extrémité libre (18B) s'étendant à partir de ladite base (20B), ladite extrémité libre (18B) présentant une surface relativement abrasive;
ledit élément élastomère (16B) comprenant (i) une partie concave supérieure (40B) qui est sensiblement lisse, et (ii) une partie convexe inférieure (50B) opposée qui comprend ladite surface relativement abrasive.

2. Instrument chirurgical (10B) selon la revendication 1, dans lequel ladite extrémité libre (18B) est effilée vers l'intérieur dans une direction s'éloignant de ladite base (20B).

3. Instrument chirurgical (10B) selon la revendication 1, dans lequel ladite surface relativement abrasive de ladite extrémité libre (18B) comprend une composition abrasive imprégnée.

4. Instrument chirurgical (10B) selon la revendication 3, dans lequel ladite composition abrasive comprend des particules de diamant.

5. Instrument chirurgical (10B) selon la revendication 3, dans lequel ladite surface relativement abrasive de ladite extrémité libre (18B) comprend une rugosification de surface.

6. Instrument chirurgical (10B) selon la revendication 1, dans lequel ladite partie de préhension (12B) comprend des sections proximale et distale agencées selon un angle obtus (α) l'une par rapport à l'autre.

7. Instrument chirurgical (10B) selon la revendication 6, dans lequel ledit angle obtus (α) est compris entre environ 120 et environ 140 degrés.

8. Instrument chirurgical (10B) selon la revendication 1, dans lequel l'élément élastomère (16B) est en silicone.

9. Procédé de fabrication d'un instrument chirurgical (10B), comprenant les étapes consistant à:
former une partie de préhension (12B) de configuration allongée et présentant des extrémités proximale et distale;
fixer une partie de pointe (14B) de façon qu'elle s'étende à partir de ladite extrémité distale de ladite partie de préhension (12B), ladite partie de pointe (14B) comprenant un élément élastomère (16B) présentant une base (20B) fixée à ladite extrémité distale de ladite partie de préhension (12B) et une extrémité libre (18B) s'étendant à partir de ladite base (20B);
assurer la présence, sur ladite extrémité libre (18B), d'une surface relativement abrasive; et
ledit élément élastomère (16B) comprenant (i) une partie concave supérieure (40B) qui est sensiblement lisse, et (ii) une partie convexe inférieure (50B) opposée qui comprend ladite surface relativement abrasive.

10. Procédé de fabrication d'un instrument chirurgical (10B) selon la revendication 9, dans lequel ladite étape consistant à assurer la présence de ladite surface relativement abrasive comprend :
une rugosification de la surface d'au moins une partie de ladite extrémité libre (18B) dudit élément élastomère (16B); ou
l'imprégnation de ladite extrémité libre (18B) avec des particules abrasives; ou
une rugosification concernant uniquement des parties latérales de ladite extrémité libre (18B).
